# FASCICULE DE BREVET EUROPEEN

(11) **EP 3 652 295 B1**
(45) Date de publication et mention de la délivrance du brevet: **16.03.2022**
(21) Numéro de dépôt: 18749039.6
(22) Date de dépôt: 12.07.2018
(51) Int. Cl.: C12M 1/33, C12M 1/00

(54) **DISPOSITIF DE FRAGMENTATION MÉCANIQUE DE TISSUS DESTINE A LA PREPARATION D'UNE COMPOSITION DE CELLULES ISOLEES, PROCEDE CORRESPONDANT**
MECHANISCHE GEWEBEFRAGMENTIERUNGSVORRICHTUNG ZUR HERSTELLUNG EINER ZUSAMMENSETZUNG VON ISOLIERTEN ZELLEN UND ENTSPRECHENDES VERFAHREN
TISSUE MECHANICAL FRAGMENTATION DEVICE INTENDED FOR THE PREPARATION OF A COMPOSITION OF ISOLATED CELLS, AND CORRESPONDING METHOD

(30) Priorité: 12.07.2017 FR 1756643
(43) Date de publication de la demande: 20.05.2020
(73) Titulaire: STEMCIS, 97490 Saint Clotilde (FR)
(72) Inventeur: ROCHE, Régis, 25115 Pouilley-les-Vignes (FR); FESTY, Franck, 97417 La Montagne (FR)
(74) Mandataire: Jacobacci Coralis Harle
(86) Numéro de dépôt international: PCT/FR2018/051763
(87) Numéro de publication internationale: WO 2019/012231

(56) Documents cités:
- WO-A1-95/09051
- WO-A1-2015/035221
- US-A1- 2005 139 704

## Description

### DOMAINE TECHNIQUE AUQUEL SE RAPPORTE L'INVENTION

La présente invention concerne de manière générale le domaine des dispositifs de préparation de compositions cellulaires. Elle concerne plus particulièrement un dispositif de fragmentation mécanique de tissus destiné à la préparation d'une composition de cellules isolées ainsi qu'un procédé utilisant le dispositif.

### ARRIERE-PLAN TECHNOLOGIQUE

Il est connu de procéder, à diverses fins, à des injections de cellules vivantes dans certaines parties du corps humain ou animal. Ces cellules possèdent des propriétés qui permettent une action sur des tissus ou des organes lésés, par la régénération ou le traitement symptomatique comme par exemple de la douleur.

Des procédés d'obtention de ces cellules ont donc été développés, notamment pour les adipocytes. En général, ces procédés permettant l'obtention de ces cellules adipocytaires possèdent une étape commune basée sur la digestion enzymatique de la matrice extracellulaire du tissu par un cocktail de protéases (collagénases) afin de libérer les cellules du tissu.

Parmi les procédés connus, on peut citer le document WO 2007/034115 A1 décrivant un procédé ainsi que des outils permettant de purifier certaines cellules contenues dans du tissu adipeux.

On connait également les procédés décrits par les documents WO 2015/035221 A1 ; US 2005/139704 A1 et WO 95/09051 A1.

Cette libération des cellules par ce procédé enzymatique est efficace mais ce type de procédé enzymatique est soumis à des contraintes réglementaires et est d'un coût important.

Pour éviter ces contraintes on peut utiliser des procédés purement mécaniques permettant de purifier les cellules adipocytaires en se passant d'enzyme. La majeure partie des procédés mécaniques connus utilisent une action mécanique afin de libérer les cellules de la structure matricielle du tissu. Les appareils permettant ces opérations sont considérés comme des Dispositifs Médicaux de classe II.

Dans le domaine de la préparation de cellules à partir de prélèvements de tissus on peut mentionner en 2014, un système StromaCell^{™} de la société Microaire qui est décrit comme une méthode semi-automatisé d'isolation mécanique des cellules du tissu adipeux avec 140 000 cellules par millilitre de lipoaspirat. Une publication de l'équipe de M. Rapisio, de la même année, décrit une isolation par vibration dans un pot pendant 6min suivit d'une centrifugation pour récupérer les cellules avec un rendement de 125 000 cellules par millilitre de lipoaspirat, donc 5% de cellules progénitrices (Aaronowitz et al. SpringerPlus 2015).

On assiste ensuite à une arrivée massive des dispositifs revendiquant l'isolation des cellules dérivées du tissu adipeux. Plus de dix systèmes sont recencés par Oberbauer en 2015 (Oberbauer et al. Cell Regeneration 2015), les plus connus étant PureGraft (Cytori), Fastem (Corios Soc Coop), Revolve/GID 700 (LifeCell Corporation/GID Group Inc), Lipogems (Lipogems International S.p.A.), StromaCell (MicroAire Surgical Instruments LLC) et MyStem (MyStem LLC). En 2015, l'équipe de Cicione décrit l'isolation cellulaire à partir de la technique MyStem EVO (Cicione et al. PRS 2015), et l'équipe de Domenis réalise une étude comparative de la technique Fastem à 2 protocoles de digestion enzymatique (Domenis et al. Stem Cell Research & Therapy 2015).

L'année d'après c'est la technique Lipogems qui est rapportées, cette technique utilise des billes dans un système clos pour microfragmenter à la main le tissu adipeux. La publication de l'équipe de Tremolada (Tremolada et al. Curr Stem Cell Rep 2016) revendique déjà plus de 7000 patients traités par ce procédé dans le monde entier dans des applications en chirurgie plastique, en chirurgie générale, en chirurgie orthopédique mais aussi en chirurgie maxillofaciale.

L'équipe de Condé-Green en 2016 réalise une revue sur l'extraction mécanique des cellules du tissu adipeux. Les études restent majoritairement in vitro et utilisent à la fois la centrifugation, l'agitation (manuelle ou électrique) et le vortex pour isoler les cellules (Condé-Green PRS 2016).

Enfin, la technique d'émulsification décrite par Tonnard en 2013 (Tonnard et al. PRS 2013) sous le nom de Nanofat qui consiste en la lyse des adipocytes par passages inter-seringues est aussi considérée comme une méthode d'extraction des cellules de la fraction stromale et notamment de cellules multipotentes (Baynard et al. PRS 2016). Le protocole Nanofat a ensuite été commercialisé par Tulip Medical.

La littérature du domaine comporte de nombreux articles dont on peut citer :
- Tonnard et al. PRS 2013 Nanofat Grafting: Basic Research and Clinical Applications
- Aronowitz et al. SpringerPlus 2015 Mechanical versus enzymatic isolation of stromal vascular fraction cells from adipose tissue
- Oberbauer et al. Cell Regeneration 2015 Enzymatic and non-enzymatic isolation systems for adipose tissue-derived cells: current state of the art
- Domenis et al. Stem Cell Research & Therapy 2015 Adipose tissue derived stem cells: in vitro and in vivo analysis of a standard and three commercially available cell-assisted lipotransfer techniques
- Cicione et al. PRS 2015 In Vitro Validation of a Closed Device Enabling the Purification of the Fluid Portion of Liposuction Aspirates
- Tremolada et al. Curr Stem Cell Rep 2016 Adipose Tissue and Mesenchymal Stem Cells: State of the Art and Lipogems® Technology Development
- Condé-Green et al. PRS 2016 Shift toward Mechanical Isolation of Adipose-derived Stromal Vascular Fraction: Review of Upcoming Techniques
- Banyard et al. PRS 2016 Phenotypic Analysis of Stromal Vascular Fraction after Mechanical Shear Reveals Stress-Induced Progenitor Populations
- Chapput et al. PRS 2016 Mechanically isolated stromal vascular fraction provides a valid and useful collagenase- free alternative technique: A comparative study
- van Dongen et al. JTERM 2017 Comparison of intraoperative procédures for isolation of clinical grade stromal vascular fraction for regenerative purposes: a systematic review.

### OBJET DE L'INVENTION

Afin de remédier aux inconvénients précités de l'état de la technique et d'autres qui apparaîtront à la lecture du présent document, la présente invention propose un mixeur de structure particulière permettant la mise en œuvre d'un procédé mécanique de préparation de cellules à partir de tissus prélevés notamment par cisaillement des tissus.

Plus particulièrement, on propose selon l'invention un dispositif de fragmentation mécanique de tissus destiné à la préparation d'une composition de cellules isolées, notamment d'adipocytes et/ou de cellules contenues dans le tissu adipeux, à partir d'un prélèvement de tissus, notamment obtenus lors d'une lipoaspiration, ladite préparation comportant au moins une étape de fragmentation mécanique des tissus dans le dispositif, ledit dispositif comportant un support motorisé et un contenant, le support motorisé comportant un moteur ayant un rotor solidaire d'un organe de couplage, le contenant comportant intérieurement un arbre rotatif pouvant être mis en rotation axiale et comportant des bras radiaux pouvant balayer l'intérieur du contenant lors de la rotation de l'arbre rotatif, le support motorisé et le contenant comportant des moyens de fixation amovible complémentaires permettant d'immobiliser le contenant sur le support motorisé, l'organe de couplage et l'arbre rotatif étant configurés pour permettre la rotation axiale de l'arbre rotatif lorsque le rotor du moteur tourne.

Selon l'invention, les bras radiaux sont de deux types : des bras de type ailette et des bras de type filaire.

D'autres caractéristiques non limitatives et avantageuses du dispositif conforme à l'invention, prises individuellement ou selon toutes les combinaisons techniquement possibles, sont les suivantes :
- le contenant est métallique,
- le contenant est en matière plastique,
- la matière plastique est polymérique,
- le contenant est translucide,
- le contenant est transparent,
- l'organe de couplage permet de coupler d'une manière amovible et en entraînement de rotation, l'arbre rotatif au rotor du moteur,
- le contenant est étanche aux liquides,
- le contenant comporte deux ouvertures, la première ouverture étant fermée par un filtre, la seconde ouverture étant fermée par un septum auto-colmatant pouvant être transpercé par une aiguille creuse ou un trocart ou une canule et se colmatant automatiquement lors du retrait de l'aiguille ou du trocart ou de la canule pour être étanche aux gaz, liquides, particules et cellules,
- le septum auto-colmatant est une valve silicone,
- la seconde ouverture est en outre fermée vers l'extérieur par un bouchon ou capuchon amovible,
- en alternative, le septum auto-colmatant est une valve ou un clapet à fermeture automatique,
- au moins une partie de l'arbre rotatif est métallique,
- au moins une partie de l'arbre rotatif est en matière plastique,
- l'arbre rotatif est métallique,
- l'arbre rotatif est en matière plastique,
- l'arbre rotatif est métallique et en matière plastique,
- les bras de type ailette sont en matière plastique ou en métal,
- les bras de type filaire sont en matière plastique, notamment en nylon ou équivalent,
- les bras de type filaire sont métalliques,
- les bras de type ailette ont une extension radiale sensiblement droite,
- les bras de type ailette ont une extension radiale incurvée,
- au moins un des bras de type ailette comporte au moins une nervure s'étendant dans un plan sensiblement perpendiculaire à son extension radiale,
- les bras de type ailette ont une section transversale choisie parmi les sections circulaire, ovale, carrée, rectangulaire, polygonale, fusiforme, en T,
- les bras de type ailette ont une section longitudinale choisie parmi les sections droite, incurvées, hélicoïdale,
- les bras de type filaire ont une section transversale choisie parmi les sections circulaire, ovale, carrée, rectangulaire, polygonale,
- les bras de type ailette sont rigides et les bras de type filaire sont flexibles,
- au moins un segment de bras de type ailette est flexible,
- au moins une partie des bras de type ailette est articulée sur l'arbre rotatif,
- les bras de type ailette et les bras de type filaire sont tous deux rigides,
- l'arbre rotatif est horizontal, le contenant est cylindrique ou tronconique droit avec une première paroi d'extrémité verticale et une seconde paroi d'extrémité verticale séparées par une paroi latérale cylindrique ou tronconique et l'arbre rotatif est coaxial à la paroi latérale,
- l'arbre rotatif est incliné par rapport à l'horizontale mais pas vertical, le contenant est cylindrique ou tronconique droit avec une première paroi d'extrémité verticale et une seconde paroi d'extrémité verticale supérieure séparées par une paroi latérale cylindrique ou tronconique,
- l'arbre rotatif est vertical, le contenant est cylindrique ou tronconique droit avec une paroi inférieure de fond, une paroi supérieure et une paroi latérale montante cylindrique ou tronconique, l'arbre rotatif étant central et coaxial à la paroi latérale, et les bras de type ailette sont disposés dans la partie inférieure de l'arbre rotatif alors que les bras de type filaire sont disposés au-dessus des bras de type ailette,
- l'arbre rotatif est d'une pièce,
- l'arbre rotatif est constitué de plusieurs pièces assemblées entre elles,
- le support comporte une partie comportant le moteur et l'organe de couplage et qui forme une partie motorisée du support,
- lorsque le contenant est fixé au support motorisé, la partie motorisée du support est disposée au-dessus du contenant, l'arbre rotatif étant entraîné par l'organe de couplage par le haut du contenant, la paroi de fond du contenant étant continue, le support motorisé ayant une forme générale notamment en U couché ou en Z afin que la partie motorisée recouvre le contenant,
- lorsque le contenant est fixé au support motorisé, la partie motorisée du support est disposée en dessous du contenant, l'arbre rotatif étant entraîné par l'organe de couplage par le bas du contenant, la paroi de fond du contenant comportant une ouverture de passage d'arbre rotatif, ladite ouverture de passage étant rendue étanche,
- l'organe de couplage assure un couplage mécanique à contact direct avec l'arbre rotatif,
- l'organe de couplage assure un couplage magnétique à distance avec l'arbre rotatif, ce qui évite une étanchéité pour l'arbre rotatif du fait que ce dernier ne traverse pas la paroi de contenant,
- le rotor du moteur est une partie de l'arbre rotatif, le couplage étant électromagnétique entre un stator du moteur dans le support motorisé et le rotor sur l'arbre rotatif du contenant, ce qui évite une étanchéité pour l'arbre rotatif du fait que ce dernier ne traverse pas la paroi de contenant,
- la paroi inférieure de fond et la paroi latérale montante sont monobloc,
- la paroi supérieure forme un couvercle du contenant,
- la paroi supérieure est rapportée sur le haut de la paroi latérale montante,
- la paroi supérieure est amovible du haut de la paroi latérale montante,
- le contenant est tronconique de diamètre 43 mm côté paroi de fond et de diamètre 54 mm côté paroi supérieure,
- la hauteur de volume utile du contenant est de 70,7 mm pour un volume utile de 100 cc,
- la hauteur hors-tout du contenant est de 97,5 mm,
- chaque bras de type ailette a au moins un bord longitudinal en biseau, ledit au moins un bord en biseau étant du côté latéral du bras balayant en premier l'intérieur du contenant lors de la rotation de l'arbre rotatif,
- le bord en biseau est un bord tranchant,
- le bord en biseau comporte en outre des indentations,
- chaque bras de type filaire est constitué d'un fil en nylon ou métallique,
- les fils en nylon ou métalliques ont un diamètre compris entre 0,5 mm et 3 mm,
- les fils en nylon ou métalliques sont étages le long de la hauteur de l'arbre rotatif, la distance entre deux étages adjacents de fil est constante selon la hauteur,
- les fils en nylon ou métalliques sont étages le long de la hauteur de l'arbre rotatif, la distance entre deux étages adjacents de fil varie selon la hauteur,
- les fils en nylon ou métalliques sont étages le long de la hauteur de l'arbre rotatif, la distance entre deux étages adjacents de fil est comprise entre 5 mm et 20 mm,
- les fils en nylon ou métalliques sont alignés selon la verticale le long de la hauteur de l'arbre rotatif,
- les fils en nylon ou métalliques sont alignés en hélice le long de la hauteur de l'arbre rotatif,
- la paroi latérale montante cylindrique ou tronconique du contenant comporte intérieurement des crantages,
- le contenant est étanche aux liquides et la paroi supérieure comporte deux ouvertures, la première ouverture étant fermée par un filtre, la seconde ouverture étant fermée par un septum auto-colmatant pouvant être transpercé par une tubulure de percement et se colmatant automatiquement lors du retrait de la tubulure de percement pour être totalement étanche,
- la tubulure de percement est une aiguille creuse ou un trocart ou une canule,
- le septum auto-colmatant est en élastomère,
- le septum auto-colmatant est une valve silicone,
- au moins une des deux ouvertures de la paroi supérieure est recouverte extérieurement d'un opercule déchirable,
- au moins une des deux ouvertures de la paroi supérieure est recouverte extérieurement d'un capuchon amovible,
- la paroi supérieure du contenant est un couvercle amovible,
- le contenant avec son arbre rotatif est à usage unique,
- le contenant avec son arbre rotatif est stérilisable,
- le support motorisé comporte un système de contrôle de la vitesse de rotation du rotor du moteur,
- le support motorisé comporte un système de mesure de la vitesse de rotation du rotor du moteur,
- le support motorisé comporte un système de mesure du couple résistant s'opposant à la rotation du rotor du moteur,
- le support motorisé comporte un système de détection de présence du contenant,
- le système de contrôle de la vitesse de rotation du rotor du moteur est configuré pour permettre au moins une des actions suivantes : rotation à direction/sens constant, rotation à direction/sens alterné, rotation à vitesse constante, rotation à vitesse variable, rotation à au moins une de plusieurs vitesses selon un programme d'évolution des vitesses ledit programme pouvant comporter des étapes conditionnelles à des résultats de mesures effectuées par un système de mesure du support motorisé,
- le support motorisé comporte une minuterie de réglage de la durée de la rotation du rotor du moteur,
- le support motorisé comporte un moyen de régulation thermique permettant de réguler la température du contenant
- de préférence, le support motorisé comporte un système de régulation de la vitesse de rotation et un temporisateur de durée de rotation préréglés, le dispositif étant automatique et l'utilisateur ne pouvant pas régler la vitesse ou la durée.

L'invention propose également un procédé de préparation d'une composition de cellules isolées, notamment d'adipocytes et/ou de cellules contenues dans le tissu adipeux, à partir d'un prélèvement de tissus, notamment obtenus lors d'une lipoaspiration, ladite préparation comportant au moins une étape de fragmentation mécanique des tissus dans un dispositif, ledit dispositif comportant un support motorisé et un contenant, le support motorisé comportant un moteur ayant un rotor solidaire d'un organe de couplage, le contenant comportant intérieurement un arbre rotatif pouvant être mis en rotation axiale et comportant des bras radiaux pouvant balayer l'intérieur du contenant lors de la rotation de l'arbre rotatif, le support motorisé et le contenant comportant des moyens de fixation amovible complémentaires permettant d'immobiliser le contenant sur le support motorisé, l'organe de couplage et l'arbre rotatif étant configurés pour permettre la rotation axiale de l'arbre rotatif lorsque le rotor du moteur tourne.

Selon ledit procédé, on met en œuvre pour la fragmentation des tissus un dispositif selon l'une quelconque des revendications précédentes et on introduit dans le contenant le tissu prélevé, on met en marche le moteur, le contenant ayant préalablement été fixé au support motorisé pour que l'organe de couplage puisse entraîner en rotation l'arbre rotatif puis, après arrêt du moteur, on récupère au moins une partie du contenu du contenant.

Dans des variantes, possiblement combinées, dudit procédé :
- la rotation de l'arbre rotatif s'effectue toujours dans le même sens,
- la rotation de l'arbre rotatif est alternée dans un sens puis l'autre,
- la rotation de l'arbre rotatif est alternée sur moins de 360°,
- la rotation de l'arbre rotatif est alternée sur plus de 360°.

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 représente une vue en coupe verticale axiale, passant par l'arbre rotatif, du contenant de l'invention,
- la figure 2 représente une vue de dessus du contenant de la figure 1,
- la figure 3 représente une vue en coupe radiale du contenant de la figure 1, et
- la figure 4 schématise les différentes étapes de préparation d'une composition de cellules isolées par utilisation du dispositif de l'invention.

### Dispositif

Dans le cadre de la solution de traitement mécanique du tissu prélevé mis en œuvre dans l'invention, la séparation des cellules du tissu est basée sur des forces d'attrition et de cisaillement que subit le tissu dans un mixeur.

Préférentiellement, ce mixeur est destiné au broyage du tissu adipeux et à l'obtention des cellules composant le tissu adipeux, principalement les cellules de la SVF (Fraction Stromal Vasculaire) mais il peut aussi servir à la purification de cellules contenues dans d'autres tissus ou organes.

Le mixeur de l'invention comporte principalement deux composants : un support comportant un moteur électrique, dit support motorisé, et un contenant adaptable sur le support motorisé. Le support motorisé comporte un moteur permettant, lorsque le contenant est fixé sur le support motorisé, de faire tourner un arbre rotatif interne du contenant, ledit arbre rotatif comportant des bras radiaux. Le contenant est fixé d'une manière amovible sur le support motorisé et un moyen de couplage amovible de l'entraînement en rotation est installé entre le support motorisé et l'arbre rotatif du contenant.

La vitesse de rotation de l'arbre rotatif est préférentiellement d'environ 4000 rpm (rotation par minute) ce qui donne un rendement de purification cellulaire optimal en termes de nombre de cellules et de viabilité. Dans des variantes, on prévoit une vitesse de rotation par minute entre 2000 et 8000 rpm et le mixeur peut comporter un moyen de réglage de la vitesse de rotation du rotor du moteur et donc de l'arbre rotatif.

L'arbre rotatif, qui est central/coaxial au contenant ici sensiblement tronconique, est muni d'ailettes radiales à sa base permettant une convection du tissu adipeux. Plus haut, le long de l'arbre rotatif, des fils en nylon ou métalliques sont fixés perpendiculairement/radialement à l'arbre rotatif afin d'assurer une coupure et une section efficaces du tissu. Le diamètre des fils en nylon ou métalliques est compris entre 0,5 mm et 3 mm. Le nombre de fil en nylon ou métalliques est compris entre 3 et 15.

Afin d'optimiser la coupure du tissu par les fils en nylon ou métalliques des crantages peuvent être insérés sur la face interne du contenant. Leur nombre peut être compris 2 et 12 et leur épaisseur peut être comprise entre 1 mm et 6 mm. La présence de ces crantages permet de créer un flux de tissu turbulent permettant un mélange optimal. Ces crantages sont néanmoins optionnels au sens où l'obtention des cellules est possible sans eux mais leur présence augmente significativement le rendement de la préparation des cellules.

Le contenant comporte à sa partie supérieure un couvercle qui comporte deux dispositifs permettant de diminuer la contamination du tissu lors de la mise en œuvre de l'invention. Le premier dispositif est un évent à travers le couvercle muni d'un filtre 0,22 µm permettant des échanges d'air dépourvus de particules infectieuses ou inertes. Le second dispositif est une valve ou un septum permettant le passage d'une canule sans passage d'air extérieur concomitamment au passage de la canule. De préférence, cette valve ou ce septum laisse passer une canule de 3 mm, canule typiquement de lipoaspiration. Ce diamètre de canule utilisable peut cependant aller de 1 mm à 5 mm. Cette valve ou ce septum permet de faire passer le tissu adipeux issu de la lipoaspiration directement dans le contenant pour broyage et de prélever le broyat après mixage dans le mixeur.

Ainsi, de préférence, le contenant est fermé par un septum qui est une valve silicone, acceptant le passage d'une canule creuse, de 1 à 5mm de diamètre, et qui se referme automatiquement lors du retrait de ladite canule. Cette valve silicone peut être complétée par un bouchon extérieur/au-dessus, pour éviter un contact direct prolongé de la valve avec l'air extérieur. Cette valve silicone non percée est étanche, notamment dans le sens de la sortie du contenant, aux liquides, particules et cellules. La valve silicone et le bouchon sont étanche aux gaz + liquide + particules + cellules dans les deux sens.

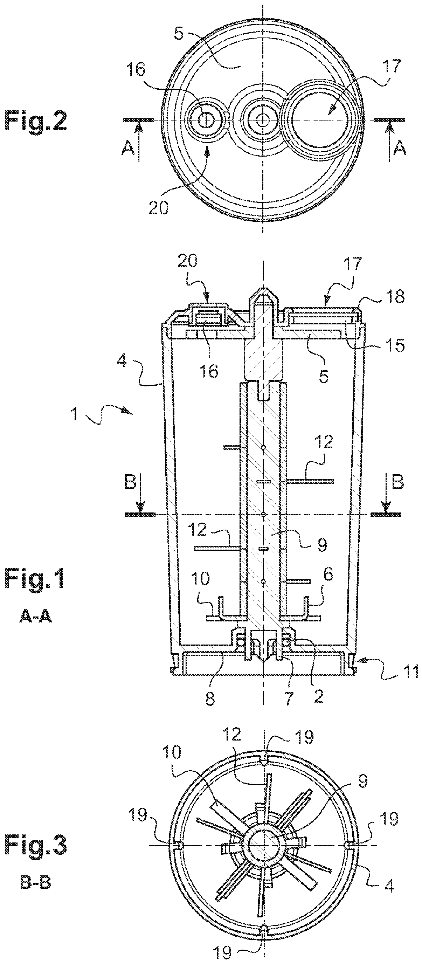

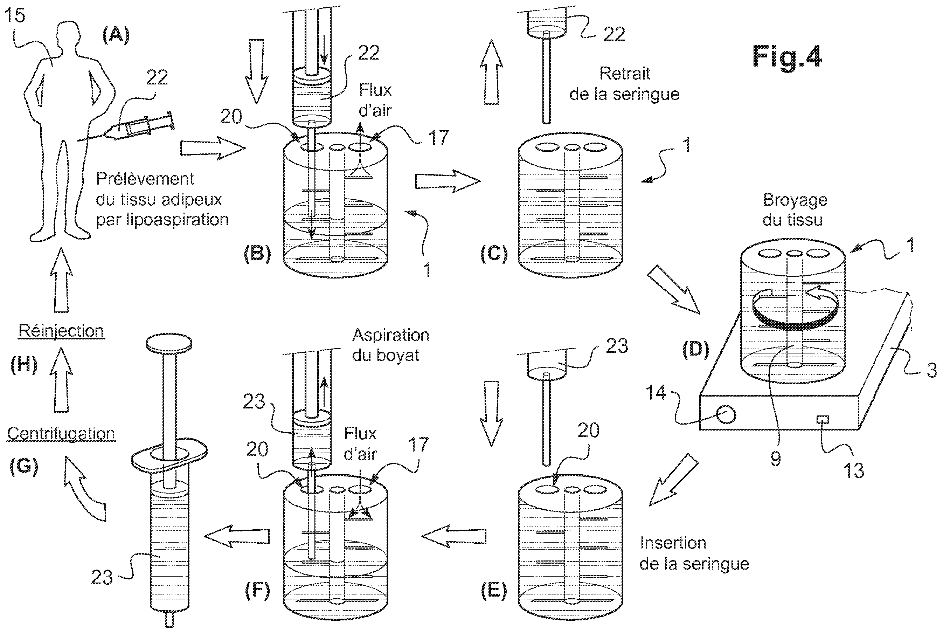

Sur les figures 1 à 3, on a représenté un exemple de contenant 1 de l'invention à utilisation verticale et actionnement de son arbre rotatif par le bas. Ce contenant est ici constitué d'un bol monobloc tronconique avec une paroi de fond 8 se poursuivant vers le haut par une paroi latérale 4 montante tronconique. La face interne de la paroi latérale 4 du contenant 1 comporte des crantages 19. L'arbre rotatif 9 est disposé central et coaxial à la paroi latérale 4 et est ici formé de l'assemblage de plusieurs pièces. L'arbre rotatif 9 traverse la paroi de fond 8 d'une manière étanche et comporte à son extrémité inférieure un moyen d'accouplement rapide 7 à un organe de couplage complémentaire (non représenté) d'un support 3 (voir figure 4) comportant un moteur électrique et sur lequel le contenant peut être fixé d'une manière amovible.

Le support motorisé 3 (voir figure 4) comporte des moyens de réglage 13, 14 de ses paramètres de fonctionnement, notamment marche/arrêt, durée et/ou vitesse de rotation. Pour permettre cette fixation amovible, des moyens de fixation 11 complémentaires de ceux du support motorisé sont mis en œuvre et dans le cas présent par vissage type quart de tour. L'étanchéité du passage de l'arbre rotatif 9 à travers la paroi de fond 8 est obtenue par mise en œuvre d'une étanchéité à joint torique 2.

Le bol tronconique à base inférieure étroite et sommet large est fermé à son sommet par une paroi supérieure 5 formant couvercle. Ce couvercle 5 est dans cet exemple clippé et n'est pas destiné à être retiré. Il peut même être soudé. Dans une variante de réalisation, ce couvercle est amovible. Le couvercle 5 comporte deux ouvertures traversantes. La première ouverture 17 est fermée par un filtre 15 de 0,22 µm et un grillage 18. La seconde ouverture 20 est fermée par une valve 16 à fermeture automatique de 3 mm de diamètre et qui assure une étanchéité totale lorsqu'elle est fermée. Ce clapet 16 s'efface lorsqu'une canule est introduite dans la seconde ouverture 20. Dans le cas d'un septum auto-colmatant du type valve silicone, cette dernière se referme après retrait de la canule. La seconde ouverture 20 est recouverte extérieurement par un capuchon amovible.

L'arbre rotatif 9 comporte des bras radiaux 10, 12 de deux types. Vers le bas de l'arbre rotatif sont disposés des bras de type ailette 10 rigides et au-dessus de ces derniers des bras de type filaire 12 flexibles ou souples en nylon ou métalliques. Les bras de type ailette 10 peuvent comporter des nervures 6 s'étendant dans un plan vertical sensiblement perpendiculaire à l'extension radiale du bras 10. Les bras radiaux, en particulier les bras filaires 12, sont étagés sur la hauteur de l'arbre rotatif 9 et répartis angulairement tout autour de ce dernier.

### Procédé

Sur la figure 4, les étapes d'obtention de la composition cellulaire souhaitée sont schématisées.

Préalablement, à l'étape A, un prélèvement de tissu adipeux par une seringue d'aspiration 22 ou un dispositif d'aspiration par dépression automatique a été effectué sur un patient 21. A l'étape B, le tissu adipeux est introduit dans le contenant 1 par passage de la canule de la seringue 22 par la seconde ouverture 20. Lors de l'injection du tissu dans le contenant, l'air mis en surpression à l'intérieur du contenant 1 s'échappe par la première ouverture 17 en traversant le filtre 15. A l'étape C la seringue 22 est retirée du contenant 1. A l'étape D, le contenant 1 a été fixé sur le support motorisé 3 et le moteur de ce dernier est activé pour entraîner en rotation l'arbre rotatif 9 du contenant 1, ce qui déstructure le tissu et libère les cellules. Les paramètres de fonctionnement sont ajustés par les moyens de réglage 13, 14 du support motorisé. A l'étape E, on vient récupérer les cellules et autres éléments du tissu qui a été déstructuré par introduction d'une canule de seringue 23 par la seconde ouverture 20. A l'étape F, lors de l'aspiration par la seringue 23, de l'air extérieur peut passer à l'intérieur du contenant par la première ouverture 17 en traversant le filtre 15. A l'étape G on procède à une centrifugation afin de sélectionner les cellules souhaitées pour une réinjection à l'étape H.

A partir d'un tissu adipeux prélevé, le mixeur de la présente invention permet d'obtenir une composition cellulaire appelée SVF (Stromal Vascular Fraction) proche de celle obtenue par le protocole utilisant de la collagénase.

Typiquement, sur le plan quantitatif, à partir de 25 ml de tissu adipeux on obtient de l'ordre de 200 000 cellules viables. Sur le plan qualitatif, l'utilisation du mixeur de l'invention permet l'obtention de l'ordre de 17% de cellules présentant un phénotype CD34+, CD31-, CD45- par rapport au nombre total de cellules purifiées regroupant les cellules considérées comme progénitrices (Mesenchymal Stem Cells) ainsi que les péricytes. Un tel ratio est très voisin de celui obtenu avec le protocole de purification basée sur l'utilisation de la collagénase (19%).

## Revendications

1. Dispositif de fragmentation mécanique de tissus destiné à la préparation d'une composition de cellules isolées, notamment d'adipocytes et/ou de cellules contenues dans le tissu adipeux, à partir d'un prélèvement de tissus, notamment obtenus lors d'une lipoaspiration, ladite préparation comportant au moins une étape de fragmentation mécanique des tissus dans le dispositif, ledit dispositif comportant un support motorisé (3) et un contenant (1), le support motorisé (3) comportant un moteur ayant un rotor solidaire d'un organe de couplage, le contenant (1) comportant intérieurement un arbre rotatif (9) pouvant être mis en rotation axiale et comportant des bras radiaux (10, 12) pouvant balayer l'intérieur du contenant (1) lors de la rotation de l'arbre rotatif (9), le support motorisé (3) et le contenant (1) comportant des moyens de fixation amovible (11) complémentaires permettant d'immobiliser le contenant (1) sur le support motorisé (3), l'organe de couplage et l'arbre rotatif (9) étant configurés pour permettre la rotation axiale de l'arbre rotatif (9) lorsque le rotor du moteur tourne,
**caractérisé en ce que** les bras radiaux (10, 12) sont de deux types : des bras de type ailette (10) et des bras de type filaire (12).

2. Dispositif selon la revendication 1, **caractérisé en ce que** les bras de type ailette (10) sont rigides et les bras de type filaire (12) sont flexibles.

3. Dispositif selon la revendication 1 ou la revendication 2, **caractérisé en ce que** l'arbre rotatif (9) est vertical, le contenant (1) est cylindrique ou tronconique droit avec une paroi inférieure (8) de fond, une paroi supérieure (5) et une paroi latérale (4) montante cylindrique ou tronconique, l'arbre rotatif (9) étant central et coaxial à la paroi latérale (4), et **en ce que** les bras de type ailette (10) sont disposés dans la partie inférieure de l'arbre rotatif (9) alors que les bras de type filaire (12) sont disposés au-dessus des bras de type ailette (10).

4. Dispositif selon l'une quelconque des revendications précédentes, **caractérisé en ce que** chaque bras de type ailette (10) a au moins un bord longitudinal en biseau, ledit au moins un bord en biseau étant du côté latéral du bras balayant en premier l'intérieur du contenant lors de la rotation de l'arbre rotatif (9).

5. Dispositif selon l'une quelconque des revendications précédentes, **caractérisé en ce que** chaque bras de type filaire (12) est constitué d'un fil en nylon ou métallique.

6. Dispositif selon la revendication 5, **caractérisé en ce que** les fils en nylon ou métalliques ont un diamètre compris entre 0,5 mm et 3 mm.

7. Dispositif selon l'une quelconque des revendications 3 à 6, **caractérisé en ce que** la paroi latérale (4) montante cylindrique ou tronconique du contenant (1) comporte intérieurement des crantages,

8. Dispositif selon l'une quelconque des revendications 3 à 7, **caractérisé en ce que** le contenant (1) est étanche aux liquides et **en ce que** la paroi supérieure (5) comporte deux ouvertures, la première ouverture (17) étant fermée par un filtre (15), la seconde ouverture (20) étant fermée par un septum auto-colmatant pouvant être transpercé par une tubulure de percement et se colmatant automatiquement lors du retrait de la tubulure de percement pour être totalement étanche.

9. Dispositif selon la revendication 8, **caractérisé en ce qu'**en alternative, le septum auto-colmatant est une valve (16) ou un clapet à fermeture automatique.

10. Procédé de préparation d'une composition de cellules isolées, notamment d'adipocytes et/ou de cellules contenues dans le tissu adipeux, à partir d'un prélèvement de tissus, notamment obtenus lors d'une lipoaspiration, ladite préparation comportant au moins une étape de fragmentation mécanique des tissus dans un dispositif, ledit dispositif comportant un support motorisé (3) et un contenant (1), le support motorisé (3) comportant un moteur ayant un rotor solidaire d'un organe de couplage, le contenant (1) comportant intérieurement un arbre rotatif (9) pouvant être mis en rotation axiale et comportant des bras radiaux (10,12) pouvant balayer l'intérieur du contenant lors de la rotation de l'arbre rotatif (9), le support motorisé (3) et le contenant (1) comportant des moyens de fixation amovible (11) complémentaires permettant d'immobiliser le contenant (1) sur le support motorisé (3), l'organe de couplage et l'arbre rotatif (9) étant configurés pour permettre la rotation axiale de l'arbre rotatif (9) lorsque le rotor du moteur tourne,
**caractérisé en ce qu'**on met en œuvre pour la fragmentation des tissus un dispositif selon l'une quelconque des revendications précédentes comportant des bras radiaux (10, 12) qui sont de deux types : des bras de type ailette (10) et des bras de type filaire (12), et qu'on introduit dans le contenant (1) le tissu prélevé, on met en marche le moteur, le contenant (1) ayant préalablement été fixé au support motorisé (3) pour que l'organe de couplage puisse entrainer en rotation l'arbre rotatif (9) puis, après arrêt du moteur, on récupère au moins une partie du contenu du contenant (1).

## Patentansprüche

1. Mechanische Gewebefragmentierungsvorrichtung zur Herstellung einer Zusammensetzung von isolierten Zellen, insbesondere von Adipozyten und/oder von im adipösen Gewebe enthaltenen Zellen, aus einer insbesondere bei einer Fettabsaugung erhaltenen Gewebeentnahme, wobei die Herstellung wenigstens einen Schritt der mechanischen Fragmentierung des Gewebes in der Vorrichtung aufweist, wobei die Vorrichtung einen motorisierten Träger (3) und einen Behälter (1) aufweist, wobei der motorisierte Träger (3) einen Motor hat, der einen mit einem Kopplungsorgan fest verbundenen Rotor aufweist, wobei der Behälter (1) innen eine drehende Welle (9) aufweist, die in axiale Drehung versetzt werden kann und radiale Anne (10, 12) aufweist, die bei Drehung der drehenden Welle (9) das Innere des Behälters (1) überstreichen können, wobei der motorisierte Träger (3) und der Behälter (1) komplementäre abnehmbare Befestigungsmittel (11) aufweisen, die ermöglichen, den Behälter (1) auf dem motorisierten Träger (3) festzulegen, wobei das Kopplungsorgan und die drehbare Welle (9) dazu ausgelegt sind, die axiale Drehung der drehbaren Welle (9) zu ermöglichen, wenn sich der Rotor des Motors dreht,
**dadurch gekennzeichnet, daß** die radialen Arme (10, 12) zweierlei Typs sind: flügelartige Anne (10) und drahtartige Arme (12).

2. Vorrichtung gemäß Anspruch 1, **dadurch gekennzeichnet, daß** die flügelartigen Arme (10) steif sind und die drahtartigen Arme (12) flexibel sind.

3. Vorrichtung gemäß Anspruch 1 oder Anspruch 2, **dadurch gekennzeichnet, daß** die drehende Welle (9) senkrecht ist, der Behälter (1) zylindrisch oder gerade kegelstumpfartig ist mit einer unteren Bodenwand (8), einer oberen Wand (5) und einer aufsteigenden zylindrischen oder geraden kegelstumpfartigen Seitenwand (4), wobei die drehende Welle (9) zur Seitenwand (4) zentral und koaxial ist, und daß die flügelartigen Arme (10) am unteren Teil der drehenden Welle (9) angeordnet sind, während die drahtartigen Anne (12) oberhalb der flügelartigen Arme (10) angeordnet sind.

4. Vorrichtung gemäß einem der vorangehenden Ansprüche, **dadurch gekennzeichnet, daß** jeder flügelartige Arm (10) wenigstens einen schrägen Längsrand hat, wobei der wenigstens eine schräge Rand auf der Seite des Arms ist, die bei Drehung der drehenden Welle (9) das Innere des Behälters zuerst überstreicht.

5. Vorrichtung gemäß einem der vorangehenden Ansprüche, **dadurch gekennzeichnet, daß** jeder drahtartige Arm (12) aus einem Nylonfaden oder einem metallenen Faden besteht.

6. Vorrichtung gemäß Anspruch 5, **dadurch gekennzeichnet, daß** die Nylonfäden oder metallenen Fäden einen Durchmesser zwischen 0,5 und 3 mm aufweisen.

7. Vorrichtung gemäß einem der Ansprüche 3 bis 6, **dadurch gekennzeichnet, daß** die aufsteigende zylindrische oder gerade kegelstumpfartige Seitenwand (4) des Behälters (1) innen Rastmittel aufweist.

8. Vorrichtung gemäß einem der Ansprüche 3 bis 7, **dadurch gekennzeichnet, daß** der Behälter (1) flüssigkeitsdicht ist und daß die obere Wand (5) zwei Öffnungen aufweist, wobei die erste Öffnung (17) durch einen Filter (15) verschlossen ist, die zweite Öffnung (20) durch ein selbstverschließendes Septum verschlossen ist, das mit einer Durchstichröhre durchstochen werden kann und sich beim Herausziehen der Durchstichröhre automatisch verschließt, um vollständig dicht zu sein.

9. Vorrichtung gemäß Anspruch 8, **dadurch gekennzeichnet, daß** das selbstverschließende Septum alternativ ein Ventil (16) oder eine automatisch schließende Klappe ist.

10. Verfahren zur Herstellung einer Komposition isolierter Zellen, inbesondere von Adipozyten und/oder von im adipösen Gewebe enthaltenen Zellen, aus einer insbesondere bei einer Fettabsaugung erhaltenen Gewebeentnahme, wobei die Herstellung wenigstens einen Schritt der mechanischen Fragmentierung des Gewebes in einer Vorrichtung aufweist, wobei die Vorrichtung einen motorisierten Träger (3) und einen Behälter (1) aufweist, wobei der motorisierte Träger (3) einen Motor hat, der einen mit einem Kopplungsorgan fest verbundenen Rotor aufweist, wobei der Behälter (1) innen eine drehende Welle (9) aufweist, die in axiale Drehung versetzt werden kann und radiale Arme (10, 12) aufweist, die bei Drehung der drehenden Welle (9) das Innere des Behälters (1) überstreichen können, wobei der motorisierte Träger (3) und der Behälter (1) komplementäre abnehmbare Befestigungsmittel (11) aufweisen, die ermöglichen, den Behälter (1) auf dem motorisierten Träger (3) festzulegen, wobei das Kopplungsorgan und die drehbare Welle (9) dazu ausgelegt sind, die axiale Drehung der drehbaren Welle (9) zu ermöglichen, wenn sich der Rotor des Motors dreht,
**dadurch gekennzeichnet, daß** für die Fragmentierung des Gewebes eine Vorrichtung gemäß einem der vorangehenden Ansprüche eingesetzt wird, die radiale Arme (10, 12) zweierlei Typs aufweist: flügelartige Anne (10) und drahtartige Arme (12), und daß in den Behälter (1) das entnommene Gewebe eingebracht wird, daß der Motor in Gang gesetzt wird, wobei der Behälter (1) vorher auf dem motorisierten Träger (3) befestigt worden ist, damit das Kopplungsorgan die drehende Welle (9) in Drehung versetzen kann, und daß nach Stillstand des Motors wenigstens ein Teil des Inhalts des Behälters (1) entnommen wird.

## Claims

1. A device for the mechanical fragmentation of tissues intended for the preparation of a composition of isolated cells, in particular adipocytes and/or cells contained in the adipose tissue, from a tissue sample, obtained in particular during a liposuction, said preparation including at least one step of mechanically fragmenting the tissues in the device, said device comprising a motorized support (3) and a container (1), the motorized support (3) comprising a motor having a rotor integral with a coupling member, the container (1) internally including an axially rotatable rotary shaft (9) and including radial arms (10, 12) capable of sweeping the inside of the container (1) as the rotary shaft (9) rotates, the motorized support (3) and the container (1) including complementary detachable fastening means (11) for immobilizing the container (1) on the motorized support (3), the coupling member and the rotary shaft (9) being configured in such a way as to allow axial rotation of the rotary shaft (9) as the motor rotor rotates,
**characterized in that** the radial arms (10, 12) are of two types: vane-type arms (10) and filamentary-type arms (12).

2. The device according to claim 1, **characterized in that** the vane-type arms (10) are rigid and the filamentary-type arms (12) are flexible.

3. The device according to claim 1 or claim 2, **characterized in that** the rotary shaft (9) is vertical, the container (1) is cylindrical or straight frustoconical in shape, with a lower bottom wall (8), an upper wall (5) and a cylindrical or frustoconical rising lateral wall (4), the rotary shaft (9) being central and coaxial to the lateral wall (4), and **in that** the vane-type arms (10) are arranged in the lower portion of the rotary shaft (9), whereas the filamentary-type arms (12) are arranged above the vane-type arms (10).

4. The device according to any one of the preceding claims, **characterized in that** each vane-type arm (10) has at least one bevelled longitudinal edge, said at least one bevelled edge being on the lateral side of the arm first sweeping the inside of the container as the rotary shaft (9) rotates.

5. The device according to any one of the preceding claims, **characterized in that** each filamentary-type arm (12) is consisted of a nylon or metal wire.

6. The device according to claim 5, **characterized in that** the nylon or metal wires have a diameter comprised between 0.5 mm and 3 mm.

7. The device according to any one of claims 3 to 6, **characterized in that** the cylindrical or frustoconical rising lateral wall (4) of the container (1) internally includes serrations.

8. The device according to any one of claims 3 to 7, **characterized in that** the container (1) is impermeable to liquids and **in that** the upper wall (5) includes two openings, the first opening (17) being closed by a filter (15), the second opening (20) being closed by a self-sealing septum which can be pierced by a piercing tubing and which seals automatically upon removal of the piercing tubing in such a way to be totally impermeable.

9. The device according to claim 8, **characterized in that**, as an alternative, the self-sealing septum is a self-closing valve (16) or flap.

10. A method for preparing a composition of isolated cells, in particular adipocytes and/or cells contained in the adipose tissue, from a tissue sample, obtained in particular during a liposuction, said preparation including at least one step of mechanical fragmentation of the tissues in a device, said device comprising a motorized support (3) and a container (1), the motorized support (3) comprising a motor having a rotor integral with a coupling member, the container (1) internally including an axially rotatable rotary shaft (9) and including radial arms (10, 12) capable of sweeping the inside of the container as the rotary shaft (9) rotates, the motorized support (3) and the container (1) including complementary detachable fastening means (11) for immobilizing the container (1) on the motorized support (3), the coupling member and the rotary shaft (9) being configured in such a way as to allow axial rotation of the rotary shaft (9) as the motor rotor rotates,
**characterized in that**, for the fragmentation of the tissues, it is implemented a device according to any one of the preceding claims that includes radial arms (10, 12) of two types: vane-type arms (10) and filamentary-type arms (12), and the sampled tissue is introduced into the container (1), the motor is powered on, the container (1) having previously been fastened to the motorized support (3) so that the coupling member can rotate the rotary shaft (9) then, after the motor has been stopped, at least one portion of the container (1) content is collected.
